(19) Europäisches Patentamt
European Patent Office
Office européen des brevets

(11) Numéro de publication: **0 019 322**
**B1**

(12) # FASCICULE DE BREVET EUROPEEN

(45) Date de publication du fascicule du brevet: **21.12.83**

(51) Int. Cl.³: **C 07 D 301/14, C 07 D 303/08**

(21) Numéro de dépôt: **80200390.5**

(22) Date de dépôt: **28.04.80**

(54) **Procédé pour la fabrication d'oxydes d'oléfines.**

(30) Priorité: **10.05.79 FR 7912145**

(43) Date de publication de la demande:
**26.11.80 Bulletin 80/24**

(45) Mention de la délivrance du brevet:
**21.12.83 Bulletin 83/51**

(84) Etats contractants désignés:
**AT BE CH DE FR GB IT LI LU NL SE**

(56) Documents cités:
**EP - A - 0 000 554**
**EP - A - 0 000 555**
**FR - A - 1 519 147**
**FR - A - 2 212 331**
**FR - A - 2 369 274**
**FR - A - 2 380 268**

(73) Titulaire: **SOLVAY & Cie (Société Anonyme)**
**Rue du Prince Albert, 33**
**B-1050 Bruxelles (BE)**

(72) Inventeur: **Walraevens, René**
**Avenue des Neuf provinces, 3**
**B-1080 Bruxelles (BE)**
Inventeur: **Lerot, Luc**
**Avenue du Centaure, 31**
**B-1200 Bruxelles (BE)**

**EP 0 019 322 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

# Procédé pour la fabrication d'oxydes d'oléfines

La présente invention concerne un procédé pour la fabrication d'oxydes d'oléfines par époxydation des oléfines correspondantes au moyen de peracides carboxyliques.

La fabrication d'oxydes d'oléfines par réaction des oléfines correspondantes avec des paracides carboxyliques en présence de solvants organiques est bien connue. Ainsi, on a proposé, dans la demande de brevet FR—A—2 212 331 (Daicel Ltd), de fabriquer du glycidol en faisant réagir de l'alcool allylique avec de l'acide peracétique. Pour éviter une décomposition du glycidol, on ajoute au mélange réactionnel, pendant ou après la réaction, un liquide dont la température d'ébullition est supérieure á celle de l'acide acétique et qui n'est pas un solvant du glycidol de manière à former deux phases lorsqu'il est mis en contact avec le glycidol. Cette technique est sans incidence sur la pureté de la solution organique de peracide et elle ne permet pas d'éviter la distillation de l'acide carboxylique avant son recyclage à la fabrication du peracide.

Dans la demande de brevet EP—A—0 000 554 (Bayer et Degussa), on propose d'époxyder des oléfines chlorées ou bromées en les faisant réagir avec des solutions de peracides carboxyliques dans un hydrocarbure chloré tel que le 1,2-dichloropropane. Lors qu'on distille le mélange réactionnel, on observe des réactions secondaires dues notamment à l'hydrolyse de l'époxyde et on est obligé de distiller tout le solvant ce qui entraîne de grosses consommations d'énergie.

Dans la demande de brevet FR—A—2 300 085 (Interox Chemicals Ltd), on propose d'utiliser pour l'époxydation une solution d'un peracide carboxylique dans un solvant organique inerte obtenue par réaction, en milieu biphasique et en présence d'un catalyseur tel que l'acide sulfurique, du peroxyde d'hydrogène avec l'acide carboxylique correspondant. Le peroxyde d'hydrogène est mis en oeuvre sous forme d'une solution aqueuse et l'acide carboxylique sous forme d'une solution dans le solvant organique inerte. Les deux solutions circulent à contre-courant. Selon la demande de brevet FR—A—2 369 274 (Propylox) qui concerne un procédé similaire, l'oléfine non transformée à l'époxydation est séparée du mélange réactionnel et absorbée dans la solution organique de peracide avant d'être recyclée au réacteur d'époxydation. Quoique beaucoup plus avantageux que les autres procédés connus, ces procédés présentent cependant encore divers inconvénients.

Ainsi, la solution organique de peracide que l'on obtient contient de l'eau et de petites quantités d'acide sulfurique. Ces impuretés sont très gênantes lors de l'époxydation ultérieure car elles favorisent la rupture du pont époxyde et la formation de produits secondaires lourds indésirables. Dès lors, il est nécessaire de soumettre la solution organique de peracide à des traitements spécifiques destinés à en éliminer ces impuretés.

Par ailleurs, la solution aqueuse appauvrie en peroxyde d'hydrogène et contenant de l'acide sulfurique dilué que l'on recueille doit être concentrée pour pouvoir récupérer le peroxyde d'hydrogène non transformé et l'acide sulfurique et les recycler. Cette concentration est très délicate à réaliser vu l'agressivité très grande des produits précités.

En outre, les solutions de peracides obtenues et destinées à être mises en oeuvre à l'époxydation sont relativement diluées. Or, il est intéressant d'utiliser à l'époxydation des solutions concentrées en peracide carboxylique pour accroître la vitesse d'époxydation.

Enfin, le mélange réactionnel obtenu à l'époxydation doit être soumis à un grande nombre de séparations en vue de récupérer séparément chacun de ses constituants.

La présente invention vise à fournir un procédé qui ne présente plus les inconvénients précités des procédés connus. En particulier, le procédé selon l'invention permet d'éliminer facilement l'eau formée lors de la fabrication du peracide, de réduire les quantités relatives de solvant inerte mises en oeuvre, et de réduire les réactions secondaires indésirables lors de l'époxydation. De plus, il conduit à un accroissement de la vitesse de réaction lors de l'époxydation. En outre, il permet de simplifier la séparation par distillation des constituants du mélange réactionnel issu de l'époxydation et d'éviter des réactions secondaires dans les bouilleurs des colonnes à distiller. Il permet de plus de travailler dans de bonnes conditions de sécurité car la concentration totale en composés peroxydés à chacune des étapes du procédé n'est pas élevée. En outre, il facilite la séparation des phases organique et aqueuse lors de la fabrication du peracide car leur pouvoir d'extraction est amélioré. Enfin il permet de réduire au minimum les pertes en réactifs, et notamment en peroxyde d'hydrogène.

L'invention concerne à cet effet un procédé pour la fabrication d'oxydes d'oléfines contenant de 3 à 10 atomes de carbone dont la température d'ébullition sous pression atmosphérique est d'au moins 370 K selon lequel

(1) on fait réagir en continu un acide carboxylique qui a une température d'ébullition supérieure à celle de l'oxyde d'oléfine, dans les mêmes conditions de pression, avec du peroxyde d'hydrogène dans un réacteur en présence d'un catalyseur et d'un solvant organique inerte;

(2) on prélève une partie du mélange réactionnel contenant le peracide carboxylique

du réacteur de l'étape (1) et on sépare la partie prélevée par décantation en une phase aqueuse et une phase organique qui constitue un solution organique de peracide carboxylique dans le solvant organique inerte;

(3) on époxyde l'oléfine correspondante dans un réacteur d'époxydation au moyen de la solution organique de peracide carboxylique obtenue à l'étape (2) de manière à obtenir un mélange réactionnel d'époxydation qui contient l'oxyde d'oléfine, l'acide carboxylique, le solvant organique inerte et l'oléfine non transformée;

(4) on sépare l'oxyde d'oléfine du mélange réactionnel quittant le réacteur d'époxydation de l'étape (3); et

(5) on recycle à l'étape (1) la solution d'acide carboxylique séparée de l'oxyde d'oléfine à l'étape (4); dans lequel

— on emploie comme solvant organique inerte un mélange de solvants qui sont inertes vis-à-vis des constituants du mélange réactionnel de fabrication du peracide carboxylique et de ceux du mélange réactionnel de l'époxydation, qui sont des solvants de l'acide carboxylique et du peracide carboxylique, qui sont très peu solubles dans l'eau et dans lesquels l'eau est très peu soluble, qui ne forment pas d'azéotrope avec l'oléfine à époxyder et avec l'oxyde d'oléfine et qui sont choisis parmi les esters carboxyliques contenant de 4 à 10 atomes de carbone, les éthers aliphatiques contenant de 4 à 12 atomes de carbone, les hydrocarbures halogénés contenant de 1 à 8 atomes de carbone, les hydrocarbures non substitués contenant de 5 à 14 atomes de carbone, les hydrocarbures substitués par des groupes nitro contenant de 3 à 8 atomes de carbone, les esters d'acide nitrique contenant de 1 à 5 atomes de carbone, les esters non acides d'acide carbonique contenant de 3 à 9 atomes de carbone et les esters non acides d'acide phosphorique contenant de 3 à 30 atomes de carbone, ce mélange comprenant: (a) de 5 à 60% en poids d'un solvant volatil dont la température d'ébullition est inférieure à celle de l'oxyde d'oléfine et ne dépasse pas 390 K, qui est capable de former avec l'eau un azéotrope hétérogène à minimum et qui ne forme pas d'azéotrope avec l'acide carboxylique; et (b) un solvant peu volatil dont la température d'ébullition est supérieure à celle de l'oléfine et de l'oxyde d'oléfine et dans lequel l'oxyde d'oléfine est soluble;

— on soumet, à l'étape (4), le mélange réactionnel d'époxydation à une série de séparations successives au cours desquelles (a) on sépare respectivement l'oléfine non transformée et le solvant organique volatil d'une solution organique contenant l'oxyde d'oléfine ainsi que le solvant organique peu volatil et l'acide carboxylique; et (b) on sépare la solution organique contenant l'oxyde d'oléfine obtenue en (4)(a) pour obtenir l'oxyde d'oléfine qui constitue la production et une solution organique d'acide carboxylique dans le solvant peu volatil; et

(6) on recycle le solvant volatil séparé à l'étape 4(a) à l'étape (1) de fabrication du peracide.

La solution organique de peracide carboxylique peut être obtenue de diverses manières connues en elles-mêmes.

Selon un mode de réalisation préféré du procédé selon l'invention, elle est fabriquée en faisant réagir en continu l'acide carboxylique correspondant avec le peroxyde d'hydrogène en présence d'un catalyseur et d'un solvant organique inerte contenant un solvant volatil capable de former avec l'eau un azéotrope hétérogène de manière à pouvoir éliminer l'eau présente dans le mélange réactionnel par distillation de l'azéotrope eau-solvant volatil. On maintient cependant dans le mélange réactionnel une quantité d'eau suffisante pour qu'il se forme une phase aqueuse distincte d'une phase organique contenant le solvant organique inerte et le peracide carboxylique. On sépare la phase aqueuse de la phase organique qui constitue la solution organique de peracide carboxylique par décantation.

Le solvant organique inerte mis en oeuvre, et qui contient le solvant volatil et le solvant peu volatil, doit être inerte vis-à-vis des constituants du mélange réactionnel lors de la fabrication du peracide carboxylique dans les conditions de cette fabrication et vis-à-vis des constituants du mélange réactionnel de l'époxydation dans les conditions de l'époxydation.

En outre, il doit dissoudre le peracide carboxylique au fur et à mesure qu'il se forme au cours de sa fabrication. De préférence, on choisit les constituants du solvant organique inerte pour que, dans les conditions de réaction, la solubilité, exprimée en mole par litre, du peracide dans la solution organique est égale à au moins 0,05.

Enfin, le solvant organique inerte est très peu soluble dans l'eau et l'eau y est très peu soluble. Des préférence, on choisit le solvant organique inerte de manière que la teneur en eau de la solution organique de peracide au cours de sa fabrication soit inférieure à la teneur en eau de l'azéotrope eau-solvant volatil dans les mêmes conditions de température et de pression. Le plus souvent, il est choisi de manière que la quantité d'eau dans la solution organique de peracide soit inférieure à 5% et de préférence inférieure à 1% en poids. Par contre, le choix de la quantité totale de solvant organique inerte dissous dans la phase aqueuse est moins important. En général, on veille à choisir un solvant

organique inerte tel qu'elle ne dépasse pas 10% et le plus souvent 5% en poids. En outre, on choisit un solvant organique inerte tel que les densités des phases aqueuse et organique obtenues lors de la fabrication du peracide soient suffisamment différentes pour en permettre la décantation.

Le solvant volatil contenu dans le solvant organique inerte répond à un certain nombre de caractéristiques. Il est capable de former avec l'eau un azéotrope hétérogène à minimum dont la température d'ébullition, dans des conditions de pression égales, est en général inférieure à la température d'ébullition des autres constituants et des autres azéotropes éventuels qui pourraient être formés dans le mélange réactionnel de fabrication du peracide. Il ne forme pas d'azéotrope avec l'acide carboxylique. Sa température d'ébullition sous pression atmosphérique ne dépasse pas 390 K. En effet les solvants ayant une température d'ébullition supérieure ne permettant pas en pratique d'éliminer azéotropiquement l'eau à la fabrication du peracide. En général, on met en oeuvre des solvants dont la température d'ébullition n'est pas inférieure à 330 K. Les solvants ayant une température d'ébullition inférieure peuvent convenir, mais sont moins intéressants d'un point de vue économique car ils doivent être mis en oeuvre en quantités beaucoup plus importantes. Le plus souvent, on utilise des solvents volatils dont la température d'ébullition est comprise entre 333 et 383 K sous pression atmosphérique. De bons résultats ont été obtenus en mettant en oeuvre des solvants volatils dont la température d'ébullition sous pression atmosphérique est comprise entre 340 et 375 K.

Le solvant organique peu volatil présent dans le solvant organique inerte répond également à un certain nombre de caractéristiques. Sa température d'ébullition est supérieure à celle de l'oléfine et de l'oxyde d'oléfine dans des conditions de pressions égales. Dans le cas où il est capable de former un azéotrope avec l'acide carboxylique, la température d'ébullition de cet azéotrope est supérieure à celles de l'oléfine et de l'oxyde d'oléfine.

Le solvant organique inerte peut éventuellement contenir plusieurs solvants organiques volatils et plusieurs solvants organiques peu volatils.

Tous les composés organiques liquides dans les conditions de réaction répondant aux conditions définies ci-avant peuvent convenir comme solvants pour réaliser le procédé selon l'invention.

Comme esters aliphatiques, alicycliques ou aromatiques d'acides mono- ou poly-carboxyliques avec des alcools mono- ou polyhydriques contenant de 4 à 10 atomes de carbone dans la molécule, on peut utiliser les formiates et acétates d'isopropyle, de propyle, de butyle, d'isobutyle, de sec-butyle, de tert-butyle, d'amyle, d'isoamyle et de sec-amyle, les mono- et di-chloroacétates, propionates, butyrates et isobutyrates de méthyle, d'éthyle, de propyle, d'isopropyle, de butyle, d'isobutyle et d'isoamyle, les valérates, isovalérates et caproates de méthyle, d'éthyle et de propyle, les acétates de méthoxyéthyle, d'éthoxyéthyle et de cyclohexyle, le pivalate de méthyle et les esters diéthyliques des acides phtalique et adipique.

Comme éthers aliphatiques symétriques ou asymétriques contenant de 4 à 12 atomes de carbone convenant bien en géneral, on peut signaler le 2,2'-dichlorodiéthyl éther, le butyléthyl éther, le tert-butyléthyl éther, le tert-amylméthyl éther, le diisopropyl éther, le dipropyl éther, le dibutyl éther, l'éthylhexyl éther et le diisobutyl éther.

Comme hydrocarbures halogénés aromatiques, aliphatiques et alicycliques contenant de 1 à 8 atomes de carbone dans leur molécule convenant bien en général, on peut signaler les hydrocarbures halogénés substitués par au moins un halogène choisi de préférence parmi le chlore, le fluor et le brome. Des hydrocarbures halogénés convenant particulièrement bien sont le tétrachlorure de carbone, le chloroforme, le chlorure de méthylène, les di-, les tri-, les tétra- et le pentachloréthanes, les trichloro-trifluoréthanes, le tri- et le tétra-chloréthylène, les mono-, les di- et les tri-chloropropanes, les butanes, méthylpropanes, pentanes et hexanes mono- ou polychlorés, le mono- et les di-chlorobenzènes, et les chlorotoluènes.

Comme hydrocarbures substitués par des groupes nitro contenant de 3 à 8 atomes de carbone aromatiques, aliphatiques, ou alicycliques convenant bien en général, on peut mentionner les nitropropanes, le nitrobenzène et le nitrocyclohexane.

Comme hydrocarbures non substitués contenant de 5 à 14 atomes de carbone aliphatiques, aromatiques, ou alicycliques convenant bien en général, on peut signaler le benzène, le toluène, le xylène, le pentane, l'hexane, l'heptane, l'octane, le diisobutyle, le cyclohexane, le méthylcyclohexane et la tétraline.

Comme esters d'acide carbonique contenant de 3 à 9 atomes de carbone dans la molécule convenant bien en général, on peut mentionner les carbonates de diméthyle, diéthyle, diisobutyle, dibutyle, di-tert-butyle, dipropyle et diisopropyle. Comme esters d'acide nitrique contenant de 1 à 5 atomes de carbone dans la molécule, on peut signaler les nitrates de méthyle, propyle butyle et isoamyle. Quant aux esters d'acide phosphorique de formule

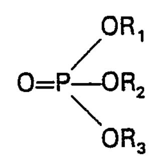

où $R_1$, $R_2$ et $R_3$ sont identiques ou différents et représentent des groupes alkyles, aryles, arylalkyles ou alkylaryles tels que la molécule contient de 3 à 30 atomes de carbone convenant bien, on peut signaler les phosphates de triméthyle, de tributyle, de trioctyle et de dioctylphényle.

Parmi les solvants pouvant convenir, on choisit avantageusement, comme solvants volatils, des solvants tels que le 1,1-dichloropropane, le 1,2-dichloropropane, le cyclohexane, le 1,2-dichloréthane, le tétrachlorure de carbone, le toluène et le benzène. De bons résultats ont été obtenus avec le 1,2-dichloropropane et le 1,2-dichloroéthane.

Comme solvant peu volatil, on peut choisir avantageusement des solvants tels que le 1,2,3-trichloropropane, les tétrachloréthanes, le tétrachloréthylène, le pentachloréthane, le 1,4-dichlorobutane, le meta-xylène, le parachlorotoluène, le chlorobenzène, le 1-nitropropane, le nitrobenzène, les chloroacétates de méthyle et d'éthyle, lacétate de butyle, le carbonate de diéthyle, le phosphate de tributyle. De bons résultats ont été obtenus avec le 1,2,3-trichloropropane et le 1,1,2,2-tétrachloréthane lorsque l'oxyde d'oléfine a une température d'ébullition inférieure à 415 K. Le 1,1,2,2-tétrachloréthane s'est révélé particulièrement adéquat.

De très bons résultats ont été obtenus lors de l'époxydation du chlorure d'allyle en épichlorhydrine en utilisant comme solvant organique inerte un mélange de 1,1,2,2-tétrachloréthane et de 1,2-dichloropropane.

La quantité relative de solvant volatil et de solvant peu volatil peut varier dans les limites précitées. De préférence, on utilise une proportion pondérale de solvant volatil inférieure à celle du solvant peu volatil. De bons résultats ont été obtenus en utilisant un mélange contenant de 5 à 35% en poids de solvant volatil.

Selon un mode de réalisation du procédé selon l'invention, on prélève en continue une partie du mélange réactionnel de fabrication du peracide, on sépare par décantation la phase aqueuse de la phase organique dans la partie prélevée et on réintroduit la phase aqueuse ainsi séparée dans le mélange réactionnel. La phase organique peut éventuellement, après avoir subi des traitements complémentaires de coalescence et de décantation, être envoyée à la réaction d'époxydation. De préférence, la phase organique, qui constitue la solution organique de peracide et qui peut contenir de petites quantités de peroxyde d'hydrogène est lavée à l'eau avant d'être envoyée à la réaction d'époxydation de manière à en éliminer le peroxyde d'hydrogène non transformé. Pour ce faire on peut utiliser de l'eau fraîche ou encore, de manière avantageuse, l'eau recueillie après condensation et décantation de l'azéotrope hétérogène eau-solvant organique volatil éliminé par distillation lors de la peracidification, ou des mélanges de ceux-ci. L'eau est en général mise en oeuvre à raison de 0,01 à 100%, et de préférence de 0,1 à 20%, du poids de la phase organique à laver. Ce lavage peut s'effectuer à des températures variables. En général, on opèra à des températures comprises entre 263 et 323 K et de préférence entre 268 et 300 K. On obtient ainsi une solution organique épurée contenant le peracide carboxylique que l'on utilise pour l'époxydation de l'oléfine et une solution aqueuse contenant du peroxyde d'hydrogène et éventuellement du catalyseur.

Selon une variante également préférée, la solution aqueuse contenant du peroxyde d'hydrogène est lavée au moyen d'au moins une partie du solvant organique volatil recueilli lors de la séparation par distillation du mélange réactionnel d'époxydation, afin d'en extraire le peroxyde d'hydrogène. La quantité de solvant volatil mise en oeuvre pour effectuer ce second lavage peut varier largement. En général, on met en oeuvre le solvant volatil à raison de 0,1 à 20 fois et de préférence de 0,2 à 10 fois le poids de solution aqueuse à traiter. Ce second lavage peut s'effectuer à des températures variables. En général on opère à des températures comprises entre 273 et 373 K et de préférence entre 280 et 323 K.

La solution de peroxyde d'hydrogène dans le solvant organique volatil ainsi obtenue est envoyée à la fabrication du peracide.

La solution aqueuse épuisée en peroxyde d'hydrogène peut, quant à elle, être soumise à divers traitements visant à recouvrir les petites quantités de solvant volatil qui pourraient y être dissoutes. On peut ainsi procéder à un entraînement à la vapeur, condenser la phase gazeuse obtenue et soumettre le liquide obtenu à une décantation pour recueillir le solvant organique volatil que l'on recycle à la fabrication du peracide.

Le solvant organique volatil contenant éventuellement du peroxyde d'hydrogène peut être prémélangé avec la solution organique d'acide carboxylique dans le solvant organique peu volatil recueillie à la distillation du mélange d'époxydation. On peut également introduire separément ces deux solutions dans le réacteur de fabrication de peracide.

Le procédé selon l'invention est appliqué à la fabrication d'oxydes d'oléfines dont la température d'ébullition sous pression atmosphérique est d'au moins 370 K. Il est appliqué à l'époxydation d'un grand nombre d'oléfines différentes comportant au moins une double liaison carbone carbone et contenant de 3 à 10 atomes de carbone au total dans leur molécule. Le procédé est appliqué de préférence aux oléfines qui contiennent des groupes $>C=CH_2$, $-CH=CH-$, ou $-CH=CH_2$.

Les alkènes ou cycloalkènes auxquels le procédé est applicable peuvent être non substitués ou substitués par un ou plusieurs substituants choisis parmi les groupes alkyles

contenant en général de 1 à 4 atomes de carbone, les groupes cycloalkyles, les groupes aryles ou les substituants contenant des hétéroatomes. Parmi les alkènes ou cycloalkènes non substitués ou substitués uniquement par des groupes alkyles, cycloalkyles ou aryles auxquels le procédé suivant l'invention s'applique, on peut citer plus particulièrement le butadiène, les pentadiènes et notamment l'isoprène, les hexènes, les hexadiènes, le diisobutylène, les octènes, les décènes, l'$\alpha$-pinène, le p.menthène, le styrène, le méthylstyrène, le vinyltoluène, les vinylcyclohexane et -hexène, le cyclohexène, le divinylbenzène et les stilbènes.

Les alkènes ou cycloalkènes utilisables dans le procédé de l'invention peuvent être également substitués par un ou plusieurs substituants contenant des hétéroatomes tels que des atomes d'halogènes et plus particulièrement des atomes de chlore, de fluor et de brome, des groupes sulfoniques ou phosphoriques, des groupes hydroxy, alkoxy, carboxy, acyl, alkoxycarbonyl, aryloxycarbonyl, acyloxy, acylamino, arylamido, alkylamido, imido ou nitrilo.

Comme dérivés halogénés insaturés utilisables, on peut citer plus particulièrement les chlorures et bromures d'allyle et de méthallyle. Parmi les alcools insaturés utilisables, on peut citer plus particulièrement l'alcool allylique, l'alcool méthallylique, et le méthylvinylcarbinol. Comme éthers insaturés convenant bien, on peut citer le méthyl allyl éther, l'éthyl allyl éther et les acétals d'acroléine. Parmi les esters insaturés convenant bien figurent les esters d'acides insaturés tels que les acides acrylique, méthacrylique et maléique, avec des alcools saturés ou insaturés, ainsi que des esters d'acides saturés avec des alcools insaturés tels que l'alcool allylique et méthallylique. Le procédé de l'invention est applicable notamment à l'acrylate de méthyle, le méthacrylate d'éthyle, le maléate de diéthyle, l'acétate d'allyle, l'acétate de méthallyle et le propionate d'allyle. Les cétones et aldéhydes insaturés peuvent également être utilisés dans le procédé de l'invention bien que dans ce dernier cas des réactions compétitives d'oxydation de la fonction aldéhyde puissent apparaître. Comme cétones ou aldéhydes utilisables, il y a lieu de signaler la méthylvinylcétone, la méthylallylcétone et l'acroléine. Parmi les amides insaturées auxquelles le procédé suivant l'invention est applicable figure l'allylacétamide. D'autres exemples d'oléfines auxquelles le procédé de l'invention est applicable sont décrits dans le brevet Etats-Unis 2 977 374 déposé le 7 mars 1958 et cédé a Union Carbide Corporation.

Le procédé selon l'invention s'applique spécialement bien à l'époxydation du cyclohexène, du butadiène, de l'alcool allylique, du styrène et, tout particulièrement, du chlorure d'allyle.

Divers types d'acides carboxyliques peuvent servir à la fabrication du peracide utilisé pour l'époxydation des oléfines dans le procédé selon l'invention. On met en oeuvre un acide carboxylique dont la température d'ébullition est supérieure à celle de l'oxyde d'oléfine dans les mêmes conditions de pression. Ces acides carboxyliques peuvent être des acides mono- ou polycarboxyliques. Dans ce dernier cas, l'acide polycarboxylique peut être mis en oeuvre dans le procédé selon l'invention également sous la forme de l'anhydride correspondant. En général, on met en oeuvre des acides carboxyliques contenant de 1 à 10 atomes de carbone tels que des acides carboxyliques aliphatiques, alicycliques ou aromatiques tels que l'acide formique, l'acide acétique, les acides chloroacétiques, l'acide propionique, l'acide butanoïque, l'acide ou l'anhydride maléique, l'acide benzoïque, l'acide cyclohexane carboxylique et les acides et anhydride phtalique. des résultats particulièrement avantageux ont été obtenus lors de la fabrication d'épichlorhydrine en mettant en oeuvre l'acide propionique.

Lors de la fabrication du peracide carboxylique, les solutions d'acide carboxylique dans le solvant organique inerte mises en oeuvre contiennent en général de 2 à 70% et de préférence de 5 à 60% en poids d'acide carboxylique. Pour préparer ces solutions, on utilise la solution d'acide carboxylique dans le solvant peu volatil récupérée lors de la distillation du mélange réactionnel d'époxydation et le solvant volatil récupéré indépendamment au cours de ces mêmes étapes de distillation, particulièrement après qu'il ait été utilisé pour récupérer le peroxyde d'hydrogène non transformé à la fabrication du peracide selon les variantes décrites ci-dessus. On peut utiliser en outre le solvant volatil provenant de la décantation de l'azéotrope distillé et des solvants frais.

Le peroxyde d'hydrogène utilisé pour la réaction peut être mis en oeuvre soit à l'état pur soit à l'état de solutions aqueuses. Lorsque le peroxyde d'hydrogène est mis en oeuvre sous la forme d'une solution aqueuse, on utilise avantageusement des solutions concentrées en peroxyde d'hydrogène contenant de 20 à 90% en poids de peroxyde d'hydrogène. D'autres concentrations peuvent également convenir mais sont moins favorables. En effet, aux concentrations plus faibles en peroxyde d'hydrogène, les quantités d'eau à éliminer par distillation azéotropique sont très importantes tandis que des solutions plus fortement concentrées en peroxyde d'hydrogène sont difficiles à produire industriellement.

Dans le mélange réactionnel de fabrication du peracide, les proportions de réactifs peuvent varier, dans l'absolu et l'une par rapport à l'autre, à l'intérieur de larges limites en fonction notamment des vitesses d'introduction choisies pour les réactifs. Ainsi, la quantité de peroxyde d'hydrogène est en général comprise entre 0,1 et 10, et de préférence entre 0,2 et 5

mole par mole de fonction carboxylique. Les résultats les plus avantageux sont obtenus d'habitude lorsqu'on introduit dans le mélange réactionnel des quantités de peroxyde d'hydrogène et d'acide carboxylique dans un rapport voisin de la stoechiométrie ou légèrement inférieure. On introduit donc de préférence le peroxyde d'hydrogène et l'acide carboxylique en quantités telles que l'on introduit entre 0,1 et 2 et de préférence entre 0,3 et 1,2 mole de peroxyde d'hydrogène par mole de fonction carboxylique.

Le peroxyde d'hydrogène peut être introduit directement dans le réacteur ou dans la solution aqueuse de catalyseur envoyée au réacteur, quand ce dernier est soluble dans l'eau. On peut ainsi introduire le peroxyde d'hydrogène dans la phase aqueuse recueillie par décantation du mélange réactionnel et recyclée en navette au réacteur. Cette introduction se fait avantageusement de façon étagée de manière à éviter des concentrations locales trop élevées en peroxyde d'hydrogène. Le débit de la navette de phase aqueuse doit être suffisant pour que la composition de la phase aqueuse enrichie en peroxyde d'hydrogène obtenue soit toujours telle que le mélange réactionnel reste en dehors de limites d'explosibilité. On peut également de façon avantageuse introduire directement le peroxyde d'hydrogène dans le réacteur.

Le catalyseur mis en oeuvre est en général un catalyseur acide convenant pour les réactions d'estérification tel que par exemple l'acide sulfurique, les acides alkyl-, aryl-, arylalkyl-, alkylaryl-sulfoniques, l'acide phosphorique, les phosphates acides d'alkyle, d'aryle, d'alkylaryle et d'arylalkyle, l'acide trifluoracétique, l'acide acétylsulfoacétique ainsi que des résines échangeuses d'ions du type polymères ou copolymères sulfonés. Comme catalyseurs préférés, on peut signaler plus particulièrement l'acide sulfurique et les acides méthane-, éthane-, benzène-, toluène-, xylène, butane-, propane-, et naphtalène-sulfoniques. Parmi ces catalyseurs, on préfère utiliser ceux qui sont solubles dans l'eau et ne sont pas, ou peu, solubles dans le liquide organique. Les meilleurs résultats sont obtenus avec les catalyseurs solubles dans l'eau dont la concentration dans la phase organique est, dans les conditions de réaction, inférieure à 3% et de préférence inférieure à 1% en poids. Des résultats particulièrement avantageux ont été obtenus avec l'acide sulfurique.

La concentration en catalyseur dans le mélange réactionnel peut varier dans de larges proportions. Pour obtenir des vitesses réactionnelles élevées, on utilise en général des concentrations en catalyseurs importantes. En général, on utilise une quantité de catalyseur supérieure à 5% du poids total d'acide et de peracide carboxyliques présents fans le mélange réactionnel. La teneur pondérale en catalyseur est de préférence comprise entre 0,1 et 30 fois le poids total d'acide et de peracide carboxyliques présents dans le mélange réactionnel. Les meilleurs résultats sont obtenus lorsque cette teneur est comprise entre 0,2 et 10 fois le poids total d'acide et de peracide carboxyliques.

Le catalyseur peut être mis en oeuvre à l'état pur. Cependant, il est avantageux de le mettre en oeuvre sous forme d'une solution aqueuse s'il soluble dans l'eau. Dans ce cas, on peut avantageusement mettre le catalyseur en oeuvre en réintroduisant dans le mélange réactionnel la phase aqueuse provenant de la décantation de celui-ci, au besoin après avoir ajouté un appoint de catalyseur. En général, la concentration en catalyseur soluble dans l'eau dans la phase aqueuse est comprise entre 10 et 60% en poids.

On maintient en général dans le mélange réactionnel une quantité d'eau suffisante pour que, dans le mélange réactionnel, le rapport pondéral de la phase aqueuse par rapport à la phase organique soit supérieur à 0,05. De préférence, ce rapport est supérieur à 0,1. Les meilleurs résultats sont obtenus lorsque ce rapport est supérieur à 0,2.

Par ailleurs, il n'y a pas intérêt, dans la plupart des cas, à maintenir dans le mélange réactionnel des quantités d'eau telles que le rapport pondéral de la phase aqueuse par rapport à la phase organique soit supérieur à 20. De préférence, ce rapport est inférieur à 10. Les meilleurs résultats sont obtenus lorsqu'il est inférieur à 5.

La phase aqueuse comprend en général de 5 à 95% et le plus souvent de 10 à 70% de son poids d'eau, le solde étant substantiellement formé par les constituants du mélange réactionnel et principalement par le peroxyde d'hydrogène et le catalyseur lorsque ce dernier est soluble et ne se présente pas sous forme de suspension solide. Elle comprend également, en général, une partie de l'acide et du peracide carboxyliques.

L'eau présente dans la phase aqueuse peut provenir notamment de la réaction ou de l'introduction de certains constituants du mélange réactionnel, en général le peroxyde d'hydrogène, et éventuellement le catalyseur, sous forme de solutions aqueuses. Elle peut aussi avoir été ajoutée intentionnellement.

La phase organique qui constitue la solution organique de peracide comprend en général de 30 à 98% et le plus souvent de 40 à 95% de son poids de solvant organique inerte, le solde étant substantiellement formé par des constituants dumélange réactionnel et principalement par l'acide carboxylique et le peracide carboxylique.

Elle peut également contenir de petites quantités de peroxyde d'hydrogène et éventuellement de catalyseur. En général la teneur en peroxyde d'hydrogène dans la phase organique ne dépasse pas 5% de son poids et sa teneur en catalyseur ne dépasse pas 1% de son poids. Le plus souvent les teneurs en

peroxyde d'hydrogène et en catalyseur de la phase organique ne dépassent pas respectivement 2 et 0,4% de son poids.

La température du mélange réactionnel est choisie en général inférieure à 373 K et, le plus souvent, est comprise entre 293 et 343 K. Des températures supérieures sont moins intéressantes car elles entraînent un risque de décomposition brutale des composés peroxydés. La pression est réglée en fonction de la température de manière à maintenir l'ébullition. Elle peut de ce fait varier dans de larges proportions. Elle est le plus souvent comprise entre 0,001 et 0,12 MPa.

La chaleur nécessaire pour maintenir l'ébullition peut être apportée selon des techniques classiques connues en elles-mêmes. On peut ainsi réchauffer le mélange réactionnel (phase aqueuse et phase organique) par mise en contact avec une surface d'échange chauffée au moyen d'un fluide caloporteur tel que la vapeur d'eau. On peut également, de façon avantageuse, introduire dans le mélange réactionnel un ou plusieurs des constituants du mélange réactionnel sous forme de vapeur. Avantageusement on introduit sous forme vapeur le solvant organique volatil provenant de la condensation et décantation de l'azéotrope eau-solvant volatil, le solvant volatil provenant de la distillation du mélange réactionnel d'époxydation éventuellement après qu'il ait servi à récupérer le peroxyde d'hydrogène de la phase organique extraite du réacteur de fabrication du peracide, ou leurs mélanges.

Pour réaliser la fabrication du peracide selon le mode de réalisation préféré, on peut utiliser n'importe quel appareillage convenant pour les mélanges réactionnels liquides et notamment les réacteurs à cuve dotés d'un système d'agitation. Plus particulièrement, il est avantageux d'utiliser des réacteurs, connus en eux-mêmes, permettant la distillation en cours de réaction d'un des constituants d'un mélange réactionnel liquide. En général, il s'agit de réacteurs permettant d'assurer un mélange intime des phases aqueuse et organique et un bon échange entre les phases liquides et la phase gazeuse de manière à favoriser la vaporisation de l'azéotrope eau-liquide organique.

Ces réacteurs sont avantageusement couplés à des colonnes de distillation, connues en elles-mêmes, telles que des colonnes à plateau ou à empilage.

Les diverses parties des réacteurs et des colonnes en contact avec le mélange réactionnel sont avantageusement réalisées dans des matériaux résistants à la corrosion tels que les aciers inoxydables, les alliages à base de nickel de marques Inconel, Hastelloy, Incoloy, Nimonic, Ni-resist, et Chlorimet, et les aciers émaillés.

La séparation par décantation du mélange réactionnel soutiré du réacteur et celle de l'azéotrope eau-liquide organique recueilli en tête de colonne peut être réalisée selon diverses techniques connues en elles-mêmes telles que décantation par gravité ou par action de la force centrifuge ou passage au travers de membranes poreuses sélectivement mouillées par l'une ou l'autre phase. Divers types d'appareils connus en eux-mêmes peuvent être utilisés à cette fin. On peut ainsi utiliser des décanteurs florentins, des décanteurs centrifuges, des filtres séparateurs à membranes ou des séparateurs électriques. La séparation par décantation peut être facilitée par une opération de coalescence des gouttelettes dans des appareils connus en eux-mêmes tels que des matelas ou des cartouches en matériaux fibreux mouillables de préférence par la phase dispersée.

Le lavage de la solution organique provenant de la décantation du mélange réactionnel de fabrication du peracide et le lavage de la solution aqueuse contenant du peroxyde d'hydrogène qui en est issu peuvent être réalisés dans divers types d'appareils connus en eux-mêmes permettant l'extraction et la décantation. On peut ainsi utiliser des colonnes d'extraction liquide-liquide fonctionnant à contre courant ou des mélangeurs décanteurs. Les décanteurs peuvent être du type de ceux signalés ci-dessus.

Pour ce qui concerne l'époxydation proprement dite, on utilise en général des rapports molaires entre le peracide carboxylique et l'oléfine à époxyder compris entre 0,01 et 20. Il est de préférence compris entre 0,05 et 10. On peut également ajouter au mélange réactionnel de petites quantités d'additifs divers tels que des inhibiteurs de polymérisation, des stabilisants du peracide, ou des séquestrants.

La réaction d'époxydation est réalisée en général à des températures comprises entre 273 K et 423 K. Celles-ci sont, de préférence, comprises entre 288 et 413 K. La pression de réaction est en général suffisante pour maintenir au moins une phase liquide. Elle est en général comprise entre 0,001 et 1 MPa. Bien entendu la température et la pression de réaction dépendent de la nature particulière de l'oléfine à époxyder.

Pour époxyder le chlorure d'allyle et l'alcool allylique, on utilise le plus souvent une température de 293 à 423 K et une pression de 0,01 et 1 MPa. Les réacteurs utilisés pour effectuer la réaction d'époxydation sont en général des réacteurs favorisant les échanges thermiques de manière à mieux contrôler les températures de réaction. On peut ainsi utiliser des réacteurs tubulaires ou des autoclaves, un réacteur unique ou des réacteurs en cascades.

Le mélange réactionnel obtenu à l'époxydation est constitué essentiellement d'oxyde d'oléfine, d'acide carboxylique, de solvant peu volatil, de solvant volatil et de réactifs non transformés; il peut éventuellement contenir de petites quantités de sous-produits et d'additifs divers. Il est soumis à des séparations préliminaires de manière à récupérer respectivement l'oléfine non transformée et le

solvant organique volatil d'une part et une solution d'oxyde d'oléfine dans le solvant organique peu volatil pouvant contenir du peracide carboxylique non transformé d'autre part.

L'oléfine recueillie est avantageusement recyclée à la réaction d'époxydation. Pour ce faire, on peut absorber l'oléfine sous forme gazeuse dans le phase organique contenant le peracide avant de l'envoyer à l'époxydation. On peut également condenser l'oléfine et ensuite seulement l'envoyer à l'époxydation. On peut aussi envoyer directement l'oléfine sous forme gazeuse dans le réacteur d'époxydation de manière à assurer, au moins partiellement l'apport calorifique nécessaire au maintien de la température réactionnelle.

La solution organique liquide d'oxyde d'oléfine et d'acide carboxylique dans la solvant organique peu volatil est ensuite soumise à une séparation, avantageusement par distillation, de façon à récupérer l'oxyde d'oléfine souhaité, d'une part et la solution d'acide carboxylique dans le solvant organique peu volatil d'autre part. L'oxyde d'oléfine peut être utilisé tel quel ou être soumis à certaines étapes d'épuration ultérieures pour en éliminer les traces éventuelles de sous-produits tels que les aldéhydes.

Divers types d'appareils connus en eux-mêmes peuvent être utilisés pour effectuer ces séparations. On peut ainsi utiliser des colonnes à distiller de tous types comportant des bouilleurs de tous types tels que des bouilleurs classiques ou des évaporateurs à films. Ces séparations se font en général par distillation. Les pressions et températures auxquelles se font ses séparations sont variables d'une séparation à l'autre et dépendent de la nature de l'oléfine du solvant volatil et de l'oxyde d'oléfine. En général pour éviter des réactions secondaires (décomposition, etc) dans les bouilleurs de colonnes à distiller, on préfère utiliser des pressions de distillation assez basses comprises le plus souvent entre 0,0005 et 0,5 MPa. Des pressions de 0,001 à 0,12 MPa conviennent bien.

Les oxydes d'oléfines obtenus selon l'invention peuvent être utilisés notamment pour la fabrication de résines.

Le procédé selon l'invention peut être réalisé en continu dans des appareils tels que ceux représentés schématiquement aux figures 1, 2 et 3 des dessins en annexe qui se rapportent à des modes de réalisation pratiques particuliers.

Selon la schéma représenté à la figure 1, le réacteur 1 est surmonté d'une colonne de distillation 2; on y introduit par la voie 3 une solution concentrée de peroxyde d'hydrogène et de catalyseur obtenue par addition de peroxyde d'hydrogène frais et éventuellement de catalyseur d'appoint respectivement par les voies 4 et 5 dans le mélangeur 6. Le mélangeur 6 est simultanément alimenté par la voie 21 par une phase aqueuse de recyclage contenant du catalyser et une peu de peroxyde d'hydrogène. Le peroxyde d'hydrogène frais peut éventuellement être introduit de façon étagée le long de cette voie (non représenté).

On introduit simultanément dans le réacteur 1 par la voie 7 une solution d'acide carboxylique dans un mélange de solvant volatil et de solvant peu volatil provenant du mélangeur 10. Le mélangeur 10 est alimenté par la voie 47 par une solution organique de recyclage contenant l'acide carboxylique, le solvant peu volatil et le solvant volatil, ce dernier pouvant être introduit séparément dans le mélangeur (non représenté). Des additions d'acide carboxylique frais par la voie 8 et de solvants frais par la voie 9 sont prévues pour compenser les pertes dans l'installation et les purges.

En cours de réaction l'azéotrope eau-solvant volatil quitte la colonne de distillation 2 par la voie 11, est condensé dans le condenseur 12 et est envoyé par la voie 13 au décanteur 14. Lorsque le solvant volatil a une densité supérieure à celle de l'eau, on recueille en tête du décanteur l'eau par la voie 15 et le solvant volatil en pied du décanteur par le voie 16; dans le cas contraire les prélèvements sont inversés.

Le solvant volatil est recyclé à la colonne de distallation 2 par la voie 16 où il constitue le reflux. Dans certains cas, on peut envoyer une partie de ce solvant volatil au mélangeur 10 où il sert de solvant de l'acide carboxylique (non représenté).

On soutire en continu du réacteur mélangeur par la voie 17 une partie du mélange réactionnel que l'on envoie au décanteur 18. Lorsque la densité de la phase organique est inférieure à celle de la phase aqueuse, on soutire en tête du décanteur 18 la phase organique qui contient le peracide et qui est envoyée dans la première zone de lavage 20 par la voie 19. En pied du décanteur 18 on recueille par la voie 21 la phase aqueuse qui est recyclée au mélangeur 6.

Dans la première zone de lavage 20, on soumet la phase organique introduite par la voie 19 à un lavage à contre courant par au moins une partie de l'eau de décantation de l'azéotrope eau-solvant volatil introduite par les voies 15 bis et 60. Une appoint d'eau fraîche par la voie 59 peut etre prevu. On recueille ainsi par la voie 21 une phase organique épurée contenant du peracide carboxylique que l'on envoie au réacteur d'époxydation 27 et par la voie 22 une solution aqueuse contenant du peroxyde d'hydrogène et éventuellement du catalyseur. Cette dernière solution pénètre par la voie 22 dans une seconde zone de lavage 23 alimentée également par la voie 24 par du solvant volatil de recyclage. On recueille ainsi par la voie 25 une solution contenant du peroxyde d'hydrogène et du solvant volatil que l'on peut recycler soit par la voie 47 au mélangeur 10, soit directement à ce même mélangeur (non représenté). La solution aqueuse épuisée en peroxyde d'hydrogène quitte la seconde zone de lavage 23 par la voie 26.

Le réacteur d'époxydation 27 est alimenté par la voie 28 en oléfine provenant du recyclage par la voie 30 et d'oléfine fraîche par la voie 29.

Le mélange réactionnel contenant notamment l'oxyde d'oléfine quitte le réacteur par la voie 31 et pénètre dans une première colonne de distillation utilisée pour le séchage 32 dont on soutire en tête un azéotrope eau-solvant volatil ou eau-oléfine selon les cas par la voie 33. On condense l'azéotrope en 34 et on l'envoie par la voie 35 au décanteur 36. S'il s'agit d'un azéotrope eau-oléfine et si la densité de l'eau est supérieure à celle de l'oléfine on recueille en pied par la voie 37 l'eau et en tête par la voie 38 l'oléfine. Selon les densités des liquides les prélèvements peuvent être inversés. Le composé organique (oléfine ou solvant volatil selon les cas) est recyclé à la colonne de distillation 32 par la voie 38 où il constitue le reflux.

Le mélange sec liquide recueilli au pied de la colonne 32 qui contient l'acide carboxylique, l'oxyde d'oléfine, le solvant volatil, le solvant peu volatil et les réactifs non transformés est envoyé par le voie 39 dans la deuxième colonne de distillation 40 d'où on soutire en tête l'oléfine si celle-ci a une température d'ébullition inférieure à celle du solvant volatil. L'oléfine est recyclée par les voies 30 et 28 au réacteur d'époxydation.

Le premier produit liquide recueilli au pied de la colonne 40 qui contient le solvant volatil, l'oxyde d'oléfine, l'acide carboxylique et le solvant peu volatil est envoyé par la voie 41 dans la troisième colonne de distillation 42 d'où on soutire en tête le solvant volatil que l'on recycle par la voie 24 à la seconde zone de lavage 23. Le deuxième produit liquide recueilli au pied de la colonne 42 qui contient le solvant peu volatil, l'oxyde d'oléfine et l'acide carboxylique est envoyé par la voie 43 dans la quatrième colonne de distillation 44 d'où on soutire en tête l'oxyde d'oléfine par la voie 45 et en pied une solution d'acide carboxylique dans le solvant peu volatil que l'on recycle par les voies 46 et 47 au mélangeur 10. L'oxyde d'oléfine peut être soumis à une distillation ultérieure pour en éliminer les traces éventuelles d'impuretés (aldéhydes...) (non représenté). Au cas où le solvant volatil a une température d'ébullition inférieure à celle de l'oléfine les colonnes 40 et 42 sont inversées. Un certain nombre de purges sont prévues dans l'installation (non représenté).

Le schéma représenté à la figure 2 est semblable à celui représenté à la figure 1 excepté pour ce qui concerne les deux zones de lavage.

La phase organique qui contient le peracide et qui est extraite du décanteur 18 est envoyée par la voie 19 dans la première zone de lavage 20 où elle est soumise à un lavage à contre courant par de l'eau fraîche introduite par la voie 48. On recueille ainsi par la voie 21 une phase organique épurée contenant du peracide carboxylique que l'on envoie au réacteur d'époxydation 27 et par la voie 22 une solution aqueuse contenant du peroxyde d'hydrogène et éventuellement du catalyseur. Cette solution est envoyée en même temps que l'eau issue du décanteur 14 par la voie 15 dans la seconde zone de lavage 23 par la voie 49. La seconde zone de lavage est simultanément alimentée par la voie 24 par du solvant volatil de recyclage. On recueille ainsi par la voie 25 une solution contenant du peroxyde d'hydrogène et du solvant volatil que l'on peut recycler par la voie 47 au mélangeur 10. La solution aqueuse épuisée en peroxyde d'hydrogène quitte la seconde zone de lavage 23 par la voie 26 et est envoyée dans la colonne d'entraînement à la vapeur 50 afin d'en éliminer les petites quantités de solvant volatil qui pourraient y être dissoutes. La colonne d'entraînement à la vapeur 50 est alimentée en vapeur par la voie 51. L'eau exempte de solvant volatil mais pouvant contenir de petites quantités de catalyseur est recueillie par la voie 52. Les vapeurs quittant la colonne 50 par la voie 53 sont condensées en 54 et envoyées par la voie 55 au décanteur 56. Le solvant volatil séparé en 56 est recueilli en 57 et peut être recyclé à la peracidification (non représenté). L'eau séparée en 56 est renvoyée par la voie 58 à la colonne 50 où elle assure le reflux.

Le schéma illustré à la figure 3 est semblable à ceux représentés aux figures 1 et 2 sauf en ce qui concerne les zones de lavage.

La phase organique qui contient le peracide et qui est soutirée en tête du décanteur 18 par la voie 19 est, éventuellement après une coalescence et une décantation supplémentaire, envoyée dans le réacteur d'époxydation 27. La phase aqueuse recueillie en tête du décanteur 14 par la voie 15 est rejetée. Le solvant volatil qui est recueilli en tête de la colonne de distillation 42 est renoyé par la voie 24 et 47 au mélangeur 10.

Afin d'illustrer l'invention, sans pour autant en limiter la portée, on donne ci-après un exemple de fabrication d'épichlorhydrine à l'intervention d'acide perpropionique.

## Exemple

L'appareil utilisé est semblable à celui schématisé à la figure 1.

Le procédé a été appliqué à la fabrication d'épichlorhydrine par époxydation du chlorure d'allyle au moyen d'acide perpropionique préparé à partir d'acide propionique.

Le liquide organique inerte utilisé comme solvant contient du 1,2-dichloropropane et du 1,1,2,2-tétrachloréthane dans un rapport pondéral 13:52.

Le réacteur de fabrication de peracide propionique est maintenu à une température de 330 K et sous une pression de 0,013 MPa.

Un système d'agitation maintient la phase aqueuse et la phase organique en émulsion.

Les deux zones de lavage sont maintenues à température ambiante.

Le réacteur d'époxydation est constitué de 5 réacteurs mélangeurs disposés en cascade et maintenu à une température d'environ 345 K sous une pression d'environ 0,101 MPa.

La composition des flux (exprimée en kg par heure) de produits dans les diverses zones de l'installation est donnée au tableau 1 ci-après. La sélectivité de la réaction d'époxydation par rapport au peroxyde d'hydrogène mis en oeuvre est voisine de 100%.

TABLEAU 1

| Voie / Constituants kg par heure | 4 | 17 | 7 | 60 | 21 | 28 | 31 | 45 |
|---|---|---|---|---|---|---|---|---|
| acide propionique | | 0,474 | 0,538 | | 0,304 | | 0,509 | |
| acide perpropionique | | 0,423 | | | 0,252 | | | |
| eau | 0,043 | 0,474 | | 0,072 | | | | |
| peroxyde d'hydrogène | 0,101 | 0,049 | | | | | | |
| acide sulfurique | | 0,534 | | | | | | |
| 1,2-dichloropropane | | 0,157 | 0,157 | | 0,157 | | 0,157 | |
| 1,1,2,2-tétrachloro-éthane | | 0,628 | 0,628 | | 0,628 | | 0,628 | |
| chlorure d'allyle | | | | | | 0,856 | 0,639 | |
| épichlorhydrine | | | | | | | 0,254 | 0,254 |
| divers | | | | | | | 0,010 | |

**Revendications**

1. Procédé pour la fabrication d'oxydes d'oléfines contenant de 3 à 10 atomes de carbone dont la température d'ébullition sous pression atmosphérique est d'au moins 370 K selon lequel

(1) on fait réagir en continu un acide carboxylique qui a une température d'ébullition supérieure à celle de l'oxyde d'oléfine, dans les mêmes conditions de pression, avec du peroxyde d'hydrogène dans un réacteur en présence d'un catalyseur et d'un solvant organique inerte;

(2) on prélève une partie du mélange réactionnel contenant le peracide carboxylique du réacteur de l'étape (1) et on sépare la partie prélevé par décantation en une phase aqueuse et une phase organique qui constitue une solution organique de peracide carboxylique dans le solvant organique inerte;

(3) on époxyde l'oléfine correspondante dans un réacteur d'époxydation au moyen de la solution organique de peracide carboxylique obtenue à l'étape (2) de manière obtenir un mélange réactionnel d'époxydation qui contient l'oxyde d'oléfine, l'acide carboxylique, le solvant organique inerte et l'oléfine non transformée;

(4) on sépare l'oxyde d'oléfine du mélange réactionnel quittant le réacteur d'époxydation de l'étape (3) et

(5) on recycle à l'étape (1) la solution d'acide carboxylique séparée de l'oxyde d'oléfine à l'étape (4);

caractérisé en ce que

— on emploie comme solvant organique inerte un mélange de solvants qui sont inertes vis-à-vis des constituants du mélange réactionnel de fabrication du peracide carboxylique et de ceux du mélange réactionnel de l'époxydation, qui sont des solvants de l'acide carboxylique et du peracide carboxylique, qui sont très peu solubles dans l'eau et dans lesquels l'eau est très peu soluble, qui ne forment pas d'azéotrope avec l'oléfine à époxyder et avec l'oxyde d'oléfine et qui sont choisis permi les esters carboxyliques contenant de 4 à 10 atomes de carbone, les éthers aliphatiques contenant de 4 à 12 atomes de carbone, les hydrocarbures halogénés contenant de 1 à 8 atomes de carbone, les hydrocarbures non substitués contenant de 5 à 14 atomes de carbone, les hydrocarbures substitués par des groupes nitro contenant de 3 à 8 atomes de carbone, les esters d'acide nitrique contenant de 1 à 5 atomes de carbone, les esters non acides d'acide carbonique contenant de 3 à 9 atomes de carbone et les esters non acides d'acide phosphorique contenant de 3 à 30 atome de carbone, ce mélange comprenant (a) de 5 à 60% en poids d'un solvant volatil dont la température d'ébullition est inférieure à celle de l'oxyde d'oléfine et ne dépasse pas 390 K, qui est capable de former avec l'eau un azéotrope hétérogène à minimum et qui ne forme pas d'azéotrope avec l'acide carboxylique; et (b) un solvant peu volatil dont la température d'ébullition est supérieure à celle de

l'oléfine et de l'oxyde d'oléfine et dans lequel l'oxyde d'oléfine est soluble;

— on soumet, à l'étape (4), le mélange réactionnnel d'époxydation à une série de séparations successives au cours desquelles (a) on sépare respectivement l'oléfine non transformée et le solvant organique volatil d'un solution organique contenant l'oxyde d'oléfine ainsi que le solvant organique peu volatil et l'acide carboxylique; et (b) on sépare la solution organique contenant l'oxyde d'oléfine obtenue en (4)(a) pour obtenir l'oxyde d'oléfine qui constitue la production et une solution organique d'acide carboxylique dans le solvant peu volatil; et

(6) on recycle le solvant volatil séparé à l'étape 4(a) à l'étape (1) de fabrication du peracide.

2. Procédé selon la revendication 1, caractérisé en ce que le solvant volatil est choisi parmi le 1,1-dichloropropane, le 1,2-dichloropropane, le cyclohexane, le 1,2-dichloréthane, le tétrachlorure de carbone, le toluène et le benzène.

3. Procédé selon la revendication 2, caractérisé en ce que le solvant volatil est le 1,2-dichloropropane.

4. Procédé selon l'une quelconque des revendications 1 à 3, caractérisé en ce que le solvant peu volatil est choisi parmi le 1,2,3-trichloropropane, les tétrachloréthanes, le tétrachloréthylène, le pentachloréthane, le 1,4-dichlorobutane, le meta-xylène, le parachlorotoluène, le chlorobenzène, le 1-nitropropane, le nitrobenzène, les chloroacétates de méthyle et d'éthyle, l'acétate de butyle, le carbonate de diéthyle, le phosphate de tributyle.

5. Procédé selon la revendication 4, caractérisé en ce que le solvant peu volatil est le 1,1,2,2-tétrachloréthane.

6. Procédé selon l'une quelconque des revendications 1 à 5, caractérisé en ce que la solution organique de peracide carboxylique est obtenue en faisant réagir l'acide carboxylique correspondant avec le peroxyde d'hydrogène en présence d'un catalyseur et du solvant organique inerte, en éliminant de l'eau présente dans le mélange réactionnel par distillation de l'azéotrope eau-solvant volatil, en maintenant dans le mélange réactionnel une quantié d'eau suffisante pour qu'il se forme une phase aqueuse distincte d'une phase organique contenant le solvant organique et le peracide carboxylique, et en séparant la phase aqueuse de la phase organique qui constitue la solution organique de peracide carboxylique.

7. Procédé selon l'une quelconque des revendications 1 à 6, caractérisé en ce que la solution organique de peracide carboxylique est, après séparation de la phase aqueuse, lavée à l'eau de manière à obtenir une solution organique épurée contenant le peracide carboxylique que l'on envoie à l'époxydation et une solution aqueuse de peroxyde d'hydrogène.

8. Procédé selon la revendication 7, caractérisé en ce que la solution aqueuse de peroxyde d'hydrogène est lavée au moyen d'au moins une partie de solvant volatil recueilli à la séparation du mélange réactionnel d'époxydation de manière à obtenir une solution aqueuse épuisée en peroxyde d'hydrogène et une solution de peroxyde d'hydrogène dans le solvant volatil que l'on recycle à la fabrication du peracide carboxylique.

9. Procédé selon l'une quelconque des revendications 1 à 8, caractérisé en ce qu'on soumet le mélange réactionnel quittant le réacteur d'époxydation à une distillation au cours de laquelle on élimine l'eau présente dans le mélange par distillation de l'azéotrope eau-solvant volatil.

10. Procédé selon l'une quelconque des revendications 1 à 9, caractérisé en ce que le procédé est appliqué à l'époxydation du cyclohexène, du butadiène, de l'alcool allylique, du styrène ou du chlorure d'allyle en l'oxyde d'oléfine correspondant.

11. Procédé selon la revendication 10, caractérisé en ce qu'il est appliqué à l'époxydation du chlorure d'allyle en épichlorhydrine.

12. Procédé selon l'une quelconque des revendications 1 à 11, caractérisé en que l'acide carboxylique est l'acide propionique.

13. Procédé selon l'une quelconque des revendications 1 à 12, caractérisé en ce que le catalyseur est l'acide sulfurique.

**Patentansprüche**

1. Verfahren zur Herstellung von Olefinoxiden enthaltend 3 bis 10 Kohlenstoffatome, deren Siedetemperatur unter Atmosphärendruck wenigstens 370 K beträgt, wobei man

(1) eine Carbonsäure, die einen Siedepunkt oberhalb dessen des Olefinoxids besitzt, kontinuierlich unter den gleichen Druckbedingungen reagieren läßt mit Wasserstoffperoxid in einem Reaktor in Anwesenheit eines Katalysators und eines organischen inerten Lösungsmittels;

(2) einen Teil des Reaktionsgemisches enthaltend die Percarbonsäure aus dem Reaktor der Stufe (1) entnimmt und den entnommenen Teil durch Dekantierung trennt in eine wässrige Phase und in eine organische Phase, die eine organische Lösung der Percarbonsäure in dem inerten organischen Lösungsmittel darstellt;

(3) das entsprechende Olefin in einem Epoxidationsreaktor epoxidiert mittels der organischen Lösung der Percarbonsäure erhalten in der Stufe (2) derart, daß man eine Epoxidations-Reaktionsmischung erhält, die das Olefinoxid, die Carbonsäure, das inerte organische Lösungsmittel und das nicht umgewandelte Olefin enthält;

(4) das Olefinoxid von dem den Epoxidationsreaktor der Stufe (3) verlassenden Reaktionsgemisch abtrennt und

(5) die in der Stufe (4) von dem Olefinoxid abgetrennte Lösung der Carbonsäure in die Stufe (1) zurückführt,

dadurch gekennzeichnet, daß,

— man als inertes organisches Lösungsmittel eine Mischung von Lösungsmitteln verwendet, welche inert sind gegenüber den Bestandteilen der Reaktionsmischung der Herstellung der Percarbonsäure und denjenigen der Reaktionsmischung der Epoxidation, welche die Lösungsmittel der Carbonsäure und der Percarbonsäure sind, welche sehr wenig löslich in Wasser sind und in denen wasser sehr wenig löslich ist, die kein Azeotrop mit dem zu epoxidierenden Olefin und mit dem Olefinoxid bilden und die ausgewählt sind unter den Carbonsäureestern enthaltend 4 bis 10 Kohlenstoffatome, den aliphatischen Äthern enthaltend 4 bis 12 Kohlenstoffatome, den halogenierten Kohlenwasserstoffen enthaltend 1 bis 8 Kohlenstoffatome, den nicht substituierten Kohlenwasserstoffen enthaltend 5 bis 14 Kohlenstoffatome, den mit Nitrogruppen substituierten Kohlenwasserstoffen enthaltend 3 bis 8 Kohlenstoffatome, den Salpetersäureestern enthaltend 1 bis 5 Kohlenstoffatome, den nicht sauren Carbonsäureestern enthaltend 3 bis 9 Kohlenstoffatome und den nicht sauren Phosphorsäureestern enthaltend 3 bis 30 Kohlenstoffatome, wobei diese Mischung umfaßt (a) 5 bis 60 Gew.-% eines flüchtigen Lösungsmittels dessen Siedepunkt unterhalb dessen des Olefinoxids liegt und 390 K nicht übersteigt, welches in der Lage ist mit Wasser wenigstens ein heterogenes Azeotrop zu bilden und welches kein Azeotrop mit der Carbonsäure bildet; und (b) ein wenig flüchtiges Lösungsmittel, dessen Siedepunkt oberhalb dessen des Olefins und des Olefinoxids liegt und in dem das Olefinoxide löslich ist;

— man in der Stufe (4) die Epoxidations-Reaktionsmischung einer Serie aufeinanderfolgender Trennungen unterwirft im Laufe wercher (a) man das nicht umgewandelte Olefin bzw. das flüchtige organische Lösungsmittel von einer organischen Lösung abtrennt, die das Olefinoxid wie auch das wenig flüchtige organische Lösungsmittel und die Carbonsäure enthält; und (b) man die in (4) (a) erhaltene, das Olefinoxid enthaltende organische Lösing abtrennt, um das Olefinoxid zu erhalten, welches das Produkt des Herstellungsverfahrens darstellt, und eine organische Lösung der Carbonsaure in dem wenig flüchtigen Lösungsmittel; und

(6) man das in Stufe 4(a) abgetrennte flüchtige Lösungsmittel in die Stufe (1) der Herstellung der Persäure zurückführt.

2. Verfahren gemäß Anspruch 1 dadurch gekennzeichnet, daß das flüchtige Lösungsmittel ausgewählt ist unter 1,1-Dichlorpropan, 1,2-Dichlorpropan, Cyclohexan, 1,2-Dichlorethan, Tetrachlorkohlenstoff, Toluol und Benzol.

3. Verfahren gemäß Anspruch 2, dadurch gekennzeichnet, daß das flüchtige Lösungsmittel 1,2-Dichlorpropan ist.

4. Verfahren gemäß einem der Ansprüche 1 bis 3, dadurch gekennzeichnet, daß das wenig flüchtige Lösungsmittel ausgewählt ist unter 1,2,3-Trichlorpropan, den Tetrachlorethanen, Tetrachloräthylen, Pentachlorethan, 1,4-Dichlorbutan, meta-Xylol, para-Chlortoluol, Chlorbenzol, 1-Nitropropan, Nitrobenzol, den Chloracetaten von Methyl und Ethyl, Butylacetat, Diethylcarbonat, Tributylphosphat.

5. Verfahren gemäß Anspruch 4, dadurch gekennzeichnet, daß das wenig flüchtige Lösungsmittel 1,1,2,2-Tetrachlorethan ist.

6. Verfahren nach einem der Ansprüche 1 bis 5, dadurch gekennzeichnet, daß die organische Lösung der Percarbonsäure erhalten wird durch Umsetzung der entsprechenden Carbonsäure mit Wasserstoffperoxid, in Anwesenheit eines Katalysators und eines inerten organischen Lösungsmittels, wobei das in der Reaktionsmischung enthaltene Wasser entfernt wird durch Destillation des Azeotrops Wasser-flüchtiges Lösungsmittel, in der Reaktionsmischung eine ausreichende Menge Wasser beibehalten wird damit sich eine wässrige Phase bildet abgesetzt gegenüber einer organischen Phase, die das organische Lösungsmittel und die Percarbonsäure enthält, und die wässrige Phase von der organischen Phase, die die organische Lösung der Percarbonsäure darstellt, abgetrennt wird.

7. Verfahren gemäß einem der Ansprüche 1 bis 6, dadurch gekennzeichnet, daß die organische Lösung der Percarbonsäure nach Abtrennung der wässrigen Phase gewaschen wird mit Wasser um eine gereinigte organische Lösung zu erhalten, die die Percarbonsäure enthält, die man zur Epoxidation führt, und eine wässrige Lösung von Wasserstoffperoxid.

8. Verfahren gemäß Anspruch 7, dadurch gekennzeichnet, daß die wässrige Lösung von Wasserstoffperoxid gewaschen wird mittels zumindest eines Teils des flüchtigen Lösungsmittels, welches gesammelt wird bei der Trennung der Reaktionsmischung der Epoxidation um so eine wässrige, an Wasserstoffperoxid verarmte Lösung und eine Lösung von Wasserstoffperoxid in dem flüchtigen Lösungsmittel zu erhalten, welche letztere man in die Fabrikation der Percarbonsäure zurückführt.

9. Verfahren gemäß einem der Ansprüche 1 bis 8, dadurch gekennzeichnet, daß man die Reaktionsmischung, die den Epoxidationsreaktor verläßt, einer Destillation unterwirft, im Laufe welcher man das in der Mischung anwesende Wasser durch Destillation des Azeotrops Wasser-flüchtiges Lösungsmittel entfernt.

10. Verfahren nach einem der Ansprüche 1 bis 9, dadurch gekennzeichnet, daß das Verfahren angewandt wird auf die Epoxidation von Cyclohexen, von Butadien, von Allylalkohol, von Styrol oder Allylchlorid zu dem jeweils entsprechenden Olefinoxid.

11. Verfahren gemäß Anspruch 10, dadurch gekennzeichnet, daß es angewandt wird auf die Epoxidation von Allylchlorid zu Epichlorhydrin.

12. Verfahren gemäß einem der Ansprüche 1 bis 11, dadurch gekennzeichnet, daß die Carbonsäure Propionsäure ist.

13. Verfahren gemäß einem der Ansprüche 1 bis 12, dadurch gekennzeichnet, daß der Katalysator Schwefelsäure ist.

**Claims**

1. Process for the manufacture of olefine oxides containing from 3 to 10 carbon atoms, the boiling point of which is at least 370 K under atmospheric pressure, wherein

(1) a carboxylic acid having a boiling point above that of the olefine oxide, under the same pressure conditions, is reacted continuously with hydrogen peroxide, in a reactor, in the presence of a catalyst and an inert organic solvent;

(2) part of the reaction mixture containing the percarboxylic acid is removed from the reactor of step (1) and the removed part is separated by decantation into an aqueous phase and an organic phase which constitutes an organic solution of percarboxylic acid in the inert organic solvent;

(3) the corresponding olefine is epoxidised in an epoxidation reactor by means of the organic solution of percarboxylic acid obtained in step (2), so as to give an epoxidation reaction mixture which contains the olefine oxide, the carboxylic acid, the inert organic solvent and the unconverted olefine;

(4) the olefine oxide is separated from the reaction mixture leaving the epoxidation reactor of step (3); and

(5) the solution of carboxylic acid separated from the olefine oxide in step (4) is recycled to step (1), characterised in that:

— the inert organic solvent employed is a mixture of solvents which are inert towards the constituents of the reaction mixture for the manufacture of the percarboxylic acid and towards the constituents of the epoxidation reaction mixture, which are solvents for the carboxylic acid and the percarboxylic acid, which are very sparingly soluble in water and in which water is very sparingly soluble, which do not form an azeotrope with the olefine to be oxidised and with the olefine oxide, and which are chosen from amongst carboxylic acid esters containing from 4 to 10 carbon atoms, aliphatic ethers containing from 4 to 12 carbon atoms, halogenohydrocarbons containing from 1 to 8 carbon atoms, unsubstituted hydrocarbons containing from 5 to 14 carbon atoms, hydrocarbons substituted by nitro groups and containing from 3 to 8 carbon atoms, nitric acid esters containing from 1 to 5 carbon atoms, non-acidic carbonic acid esters containing from 3 to 9 carbon atoms, and non-acidic phosphoric acid esters containing from 3 to 30 carbon atoms, this mixture comprising: (a) from 5 to 60% by weight of a volatile solvent, the boiling point of which is lower than that of the olefine oxide and does not exceed 390 K, which is capable of forming, with water, a heterogeneous azeotrope with a minimum, and which does not form an azeotrope with the carboxylic acid; and (b) a solvent of low volatility, the boiling point of which is higher than that of the olefine and the olefine oxide, and in which the olefine oxide is soluble, and

— the epoxidation reaction mixture is subjected in step (4), to a series of successive separations during which: (a) the unconverted olefine and the volatile organic solvent are respectively separated from an organic solution containing the olefine oxide together with the organic solvent of low volatility and the carboxylic acid; (b) the organic solution containing the olefine oxide, obtained in (4)(a), is separated to give the olefine oxide, which constitutes the production, and an organic solution of carboxylic acid in the solvent of low volatility; and (c) the volatile solvent separated off in step 4(a) is recycled to step (1) for the manufacture of the peracid.

2. Process according to Claim 1, characterized in that the volatile solvent is chosen from amongst 1,1-dichloropropane, 1,2-dichloropropane, cyclohexane, 1,2-dichloroethane, carbon tetrachloride, toluene and benzene.

3. Process according to Claim 2, characterised in that the volatile solvent is 1,2-dichloropropane.

4. Process according to any one of Claims 1 to 3, characterised in that the solvent of low volatility is chosen from amongst 1,2,3-trichloropropane, tetrachloroethanes, tetrachloroethylene, pentachloroethane, 1,4-dichlorobutane, meta-xylene para-chlorotoluene, chlorobenzene, 1-nitropropane, nitrobenzene, methyl and ethyl chloroacetate, butyl acetate, diethyl carbonate and tributyl phosphate.

5. Process according to Claim 4, characterised in that the solvent of low volatility is 1,1,2,2-tetrachloroethane.

6. Process according to any one of Claims 1 to 5, characterised in that the organic solution of percarboxylic acid is obtained by reacting the corresponding carboxylic acid with hydrogen peroxide, in the presence of a catalyst and the inert organic solvent, by removing water present in the reaction mixture by distillation of the

water/volatile solvent azeotrope, by keeping a sufficient amount of water in the reaction mixture to allow the formation of an aqueous phase which is distinct from an organic phase containing the organic solvent and the percarboxylic acid, and by separating the aqueous phase from the organic phase which constitutes the organic solution of percarboxylic acid.

7. Process according to any one of Claims 1 to 6, characterised in that, after separating off the aqueous phase, the organic solution of percarboxylic acid is washed with water so as to give a purified organic solution containing the percarboxylic acid, which solution is sent to the epoxidation, and an aqueous solution of hydrogen peroxide.

8. Process according to Claim 7, characterised in that the aqueous solution of hydrogen peroxide is washed with at least part of the volatile solvent collected on separation of the epoxidation reaction mixture, so as to give an aqueous solution exhausted of hydrogen peroxide and a solution of hydrogen peroxide in the volatile solvent, which latter solution is recycled to the manufacture of the percarboxylic acid.

9. Process according to any one of Claims 1 to 8, characterised in that the reaction mixture leaving the epoxidation reactor is subjected to distillation, during which the water present in the mixture is removed by distillation of the water/volatile solvent azeotrope.

10. Process according to any one of Claims 1 to 9, characterised in that it is applied to the epoxidation of cyclohexene, butadiene, allyl alcohol, styrene or allyl chloride to give the corresponding olefine oxide.

11. Process according to Claim 10, characterised in that it is applied to the epoxidation of allyl chloride to give epichlorohydrin.

12. Process according to any one of Claims 1 to 11, characterised in that the carboxylic acid is propionic acid.

13. Process according to any one of Claims 1 to 12, characterised in that the catalyst is sulphuric acid.

FIG 1

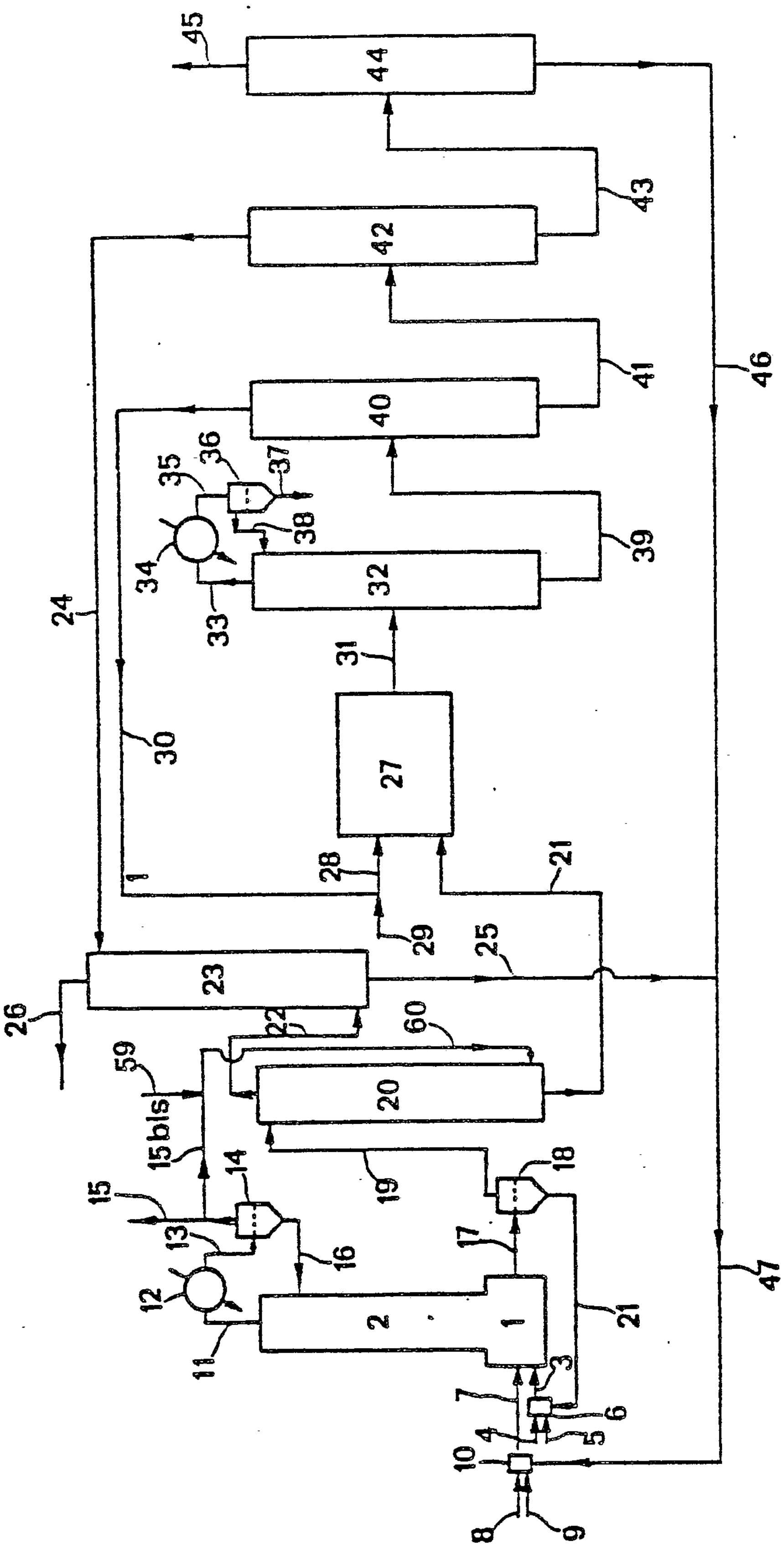

1
2
3
4
5
6
7
8
9
10
11
12
13
14
15
15 bis
16
17
18
19
20
21
22
23
24
25
26
27
28
29
30
31
32
33
34
35
36
37
38
39
40
41
42
43
44
45
46
47
59
60

FIG 2

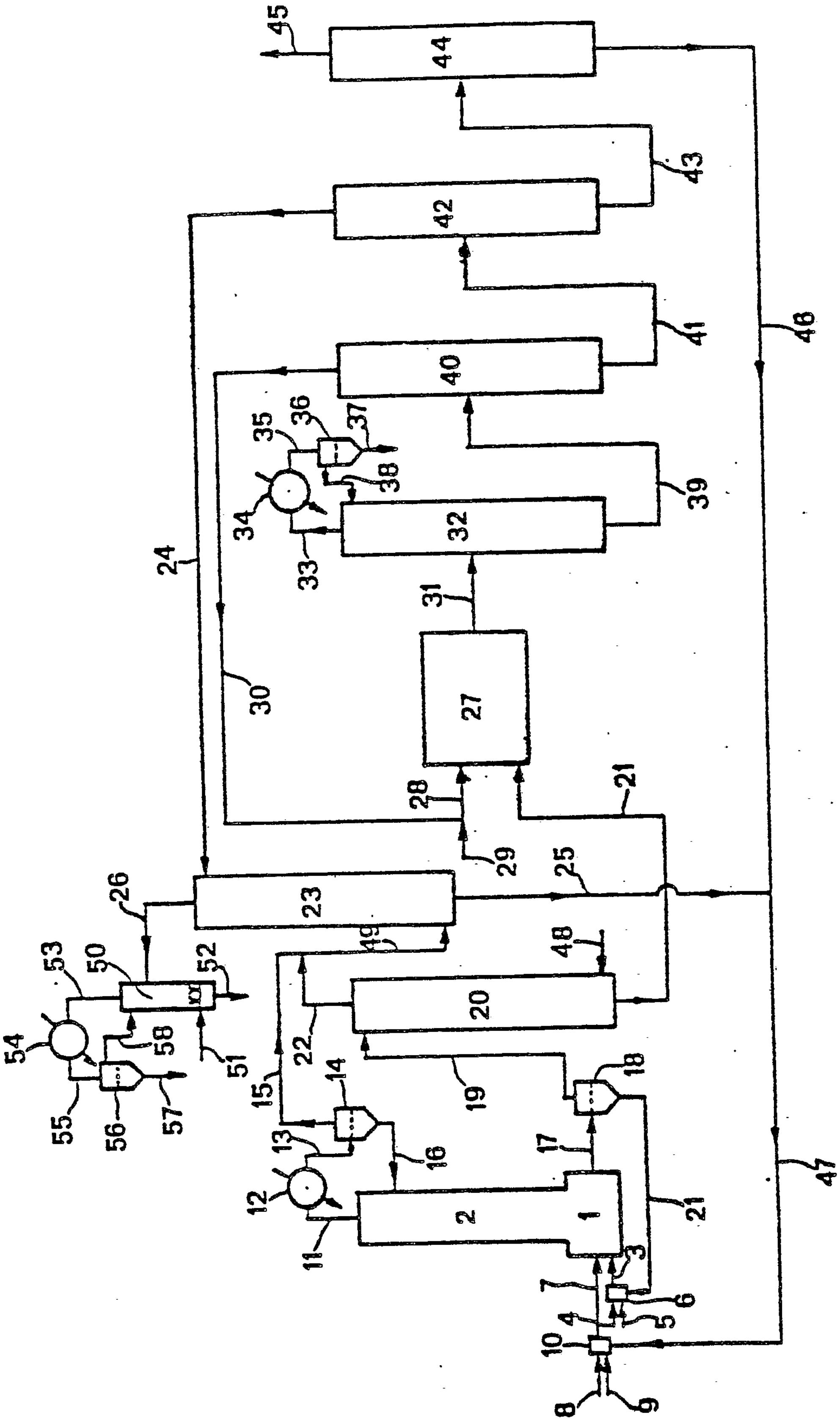

FIG 3

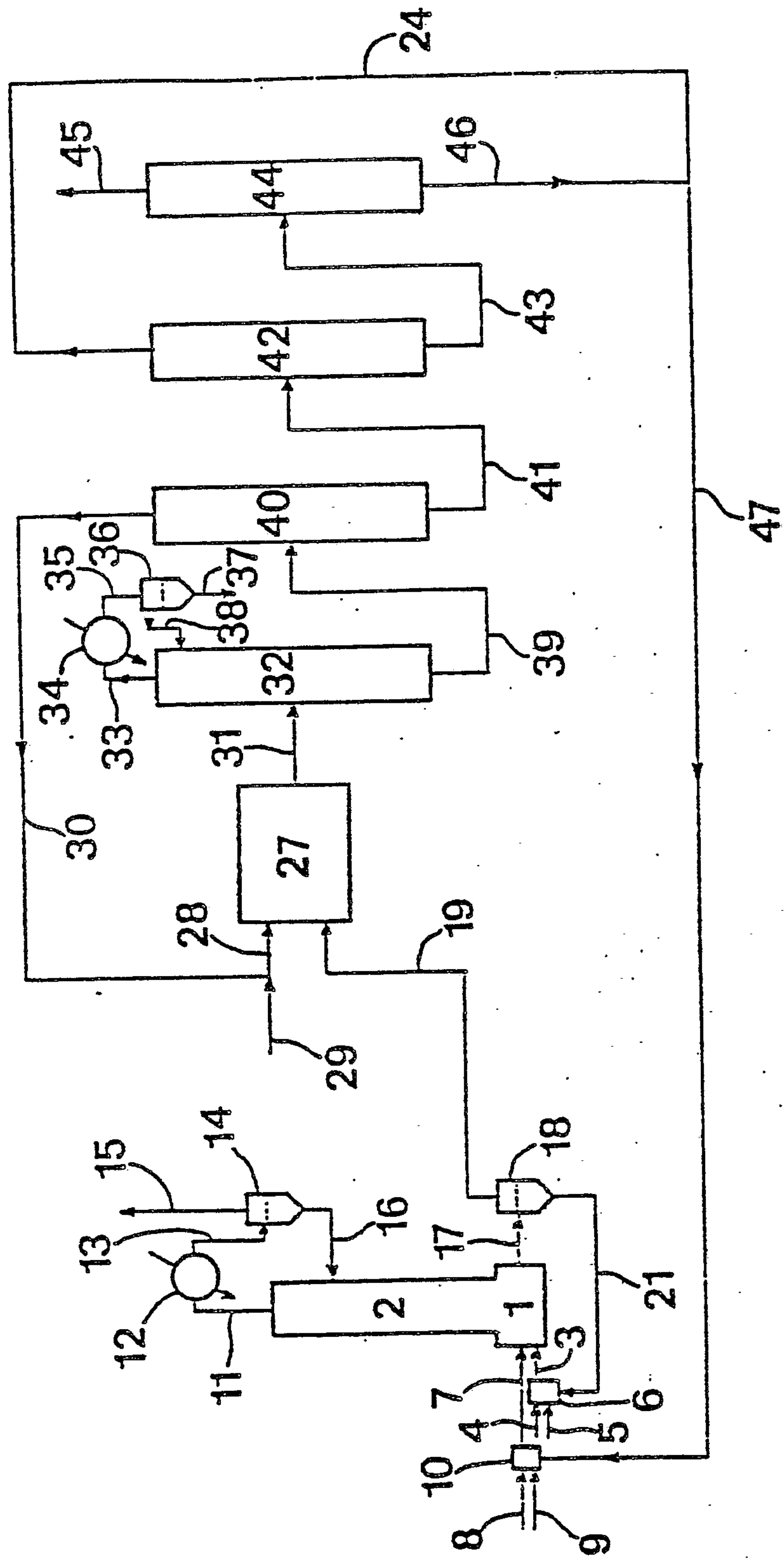

1
2
3
4
5
6
7
8
9
10
11
12
13
14
15
16
17
18
19
21
24
27
28
29
30
31
32
33
34
35
36
37
38
39
40
41
42
43
44
45
46
47